# EUROPÄISCHE PATENTSCHRIFT

(11) **EP 1 752 113 B1**
(45) Veröffentlichungstag und Bekanntmachung des Hinweises auf die Patenterteilung: **10.12.2008**
(21) Anmeldenummer: 05017419.2
(22) Anmeldetag: 10.08.2005
(51) Int. Cl.: A61F 2/90

(54) **Rohrförmige Stützprothese mit sich seitlich überlappenden Krümmungsbögen**
Tubular stent with laterally overlapping arcs
Stent tubulaire avec courbures chevauchantes

(43) Veröffentlichungstag der Anmeldung: 14.02.2007
(73) Patentinhaber: Axetis AG, 6300 Zug (CH)
(72) Erfinder: Fliedner, Thilo, Dr. OPTIRAY Medizintechnik GmbH, 80939 München (DE)
(74) Vertreter: Grünecker, Kinkeldey, Stockmair & Schwanhäusser Anwaltssozietät

(56) Entgegenhaltungen:
- EP-A- 0 790 041
- DE-A1- 10 123 441
- US-A1- 2003 144 729
- US-A1- 2004 102 831
- US-A1- 2004 127 972
- US-A1- 2005 043 778
- US-B1- 6 620 201

## Beschreibung

Die vorliegende Erfindung bezieht sich auf eine rohrförmige Stützprothese, insbesondere einen Gefäßstent, für Gefäße oder intrakorporale Lumina mit mindestens zwei nebeneinander angeordneten und an mindestens einer Verbindungsstelle miteinander verbundenen, expandierbaren Stützringen, die jeweils von einem mäanderförmig zu mehreren Krümmungsbögen gewundenen Filament gebildet sind.

Eine solche Stützprothese ist z.B. aus der DE 295 21 206 U1 bekannt. Der dort beschriebene sogenannte "Stent" weist mehrere nebeneinander angeordnete Stützringe auf, die jeweils aus einem gleichmäßigen, zu einem Mäandermuster geformten Filament bestehen. Einige der Krümmungsbögen bzw. Schlaufen des Mäandermusters eines Stützrings sind über ebenfalls schlaufenförmig ausgeformte Verbinder mit zugeordneten Krümmungsbögen eines benachbarten Stützrings verbunden. Aufgrund der Schlaufenform der Verbinder, verlängern sich diese beim Aufdehnen des Gefäßstents, so dass sich die durch das Aufdehnen der Stützringe einstellende Längenschrumpfung wieder kompensiert wird und der Gefäßstent im wesentlichen seine ursprüngliche Länge beibehält. Die Mäanderform und die Form und Anordnung der Verbinder sind so gewählt, dass sich die einzelnen Krümmungsbögen bzw. Schlaufen beim Aufdehnen des Gefäßstents nicht aufstellen ("flaring"), was zu einer unnötigen Verletzung der Gefäßwandung führen könnte. Üblicherweise werden die Gefäßstents mittels eines Katheters an den Zielort gebracht, wo sie mit Hilfe eines Ballons (Ballonkatheter) im Gefäß durch Dehnung der miteinander verbundenen Stützringe auf das notwendige Lumen und den notwendigen Durchmesser zur Abstützung des Gefäßes expandiert werden.

Bei vorbekannten Stents ist es jedoch schwierig, die verschiedenen Anforderungen durch ein geeignetes Ausformen der Stützringe und der Verbinder gleichzeitig zu erfüllen. Maßnahmen, die eine minimale Längsschrumpfung beim Aufdehnen bewirken, führen nicht immer zu einer gewünschten Stabilität gegen Aufrichten der Krümmungsbögen der Mäanderform. Darüber hinaus sind Gefäßstents oftmals mit Beschichtungen versehen, die die Verträglichkeit verbessern sollen. Solche Beschichtungen müssen zum Teil extreme Verformungen beim Aufdehnen des Gefäßstents mitmachen können.

Aus der US 6,620,201 B1 sind Netzmuster einer rohrförmigen Stützprothese bekannt. Es besteht aus mehreren miteinander verbundenen expandierbaren Stützringen, welche aus jeweils einem zick-zack-förmig mäandernden Filament geformt sind. Nebeneinander liegende mäanderförmige Bänder werden über erste und zweite einzelne bzw. getrennte Strukturelemente miteinander verbunden. Das zweite Strukturelement weist eine S-Form auf und das dritte Strukturelement weist im Wesentlichen in seiner Mitte eine abgerundete Schulter auf.

Aus der US 2003/144,729 A1 ist ein Stent bekannt, bei dem die einzelnen Stützringe selbst aus zwei mäanderförmigen Bändern gebildet sind, die sich teilweise im Verbindungsbereich überlappen. Die einzelnen Stützringe sind mit geschwungenen oder geraden Verbindern (100) miteinander verbunden.

Die US 2004/127,972 A1 befasst sich mit einem Stent, der zueinander angeordnete Stützringe aufweist, die über im Wesentlichen gerade Verbindungsstücke miteinander verbunden sind. Die Stützringe selbst bestehen aus zwei mäanderförmigen Bändern, die sich teilweise überlappen.

Aufgabe der Erfindung ist nunmehr, eine rohrförmige Stützprothese der eingangs genannten Art bereitzustellen, die verbesserte Eigenschaften hinsichtlich verminderter Aufrichtneigung der Krümmungsbögen, bei bevorzugt weitgehender Vermeidung von Spannungsspitzen, aufweist.

Diese Aufgabe wird erfindungsgemäß durch eine rohrförmige Stützprothese nach Anspruch 1 gelöst. Mindestens ein Krümmungsbogen eines ersten Stützrings und ein Krümmungsbogen eines benachbarten zweiten Stützrings überlappen sich in Umfangsrichtung seitlich, wobei im Überlappungsbereich die Verbindungsstelle gebildet ist.

Bislang sind die Stützringe meist nebeneinander angeordnet worden und haben sich allenfalls im Bereich einiger Krümmungsscheitel zur Herstellung einer Verbindung berührt. Meistens sind sie jedoch beabstandet und durch speziell ausgeformte Verbinder miteinander verbunden. Die Überlappung ist erfindungsgemäß jeweils auf den kürzesten Abstand zweier umlaufender Mittellinien von den benachbarten Stützringen bezogen. Die Gesamtbreite eines Stützrings (senkrecht zu dieser Mittellinie gemessen) muss demnach größer sein als der Abstand dieser beiden erwähnten Mittellinien. Durch diese Definition soll eine scheinbare Überlappung bei schräg angeordneten bzw. elliptisch umlaufenden Stützringen ausgeschlossen sein. Die Überlappung sorgt dafür, dass die benachbarten Stützringe quasi ineinander verzahnt sind und sich die Krümmungsbögen an den Verbindungsstellen im Krümmungsbereich gegenseitig an einem Aufstellen der Krümmungsbögen während des Expansionsvorgangs hindern. Des Weiteren können durch eine solche Maßnahme Spannungsspitzen im Verbindungsbereich gegenüber herkömmlichen Gefäßstents verringert werden, so dass dieser Aufbau auch für Beschichtungen sehr gut geeignet ist. Bevorzugterweise werden die Gefäßstents aus einem Rohrrohling hergestellt, in dem die mäanderförmig ausgestalteten Stützringe bzw. dass diese bildende Filament aus dem Vollmaterial herausgelasert und anschließend elektropoliert werden bzw. wird. Durch ein solches Herstellverfahren können äußerst filigrane, netzartige Strukturen erzeugt werden.

Die sich überlappenden Krümmungsbögen weisen im Überlappungsbereich einen gemeinsamen Filamentabschnitt auf, der die Verbindungsstelle bildet. Der Verbinder ist demnach Teil der Krümmungsbögen selbst, und zwar dort, wo sich diese überlappenden Krümmungsbögen berühren bzw. ineinander übergehen. Der Überlappungsbereich übt dann beim Aufdehnen des Gefäßstents unmittelbaren Einfluss auf die Verformung der Krümmungsbögen selbst aus. Bislang verwendete Verbinder sollten ihre eigenen Verformungseigenschaften möglichst unabhängig von den Krümmungsbögen entfalten oder dienten lediglich der Fixierung zweier benachbarter Krümmungsbögenscheitelpunkte zueinander. Durch die erfindungsgemäße Ausgestaltung entsteht ein viel komplexerer Verformungsvorgang, der jedoch letztendlich zu einer Verbesserung des Aufdehnverhaltens des Gefäßstents führt.

Damit die Verbindungsstellen möglichst einer Schrumpfung der Länge des Gefäßstents beim Expansionsvorgang entgegenwirken, ist vorgesehen, dass der gemeinsame Filamentabschnitt im Überlappungsbereich in einem Winkel α von größer 0°, bevorzugt größer 10°, relativ zu einer Parallelen der Stützprothesenhauptachse geneigt ist. Die Neigung sorgt dafür, dass der Verbindungsbereich sich beim Aufdehnen des Gefäßstents verschwenkt, insbesondere sich auf einen Winkel im Bereich von 90° zubewegt. Hierdurch wird einer Längenreduktion entgegengewirkt.

Darüber hinaus ist vorgesehen, dass die gemeinsamen Filamentabschnitte der Überlappungsbereiche auf der einen Stirnseite eines Stützrings in die entgegengesetzte Richtung zu den Überlappungsbereichen der anderen Stirnseite desselben Stützrings geneigt sind. Die Überlappungsbereiche auf den gegenüberliegenden Stirnseiten eines Stützrings schwenken quasi beim Expansionsvorgang aufeinander zu, so dass ein Teil der Expansionsspannungen wieder abgebaut wird.

Eine Ausführungsform sieht vor, dass eine umlaufende Ringmittellinie eines Stützrings im wesentlichen in einer senkrecht zur Stützprothesenhauptachse verlaufenden Querschnittsebene angeordnet ist. Die umlaufende Ringmittellinie ist mittig zwischen den beiden Stirnseiten eines Stützrings angeordnet, so dass zumindest die Scheitelpunkte der sich mit anderen Krümmungsbögen benachbarter Stützringe überlappenden Krümmungsbögen einen gleich weiten Abstand zu dieser Mittellinie haben. Wenn diese Mittellinie in einer senkrecht zur Stützprothesenhauptachse verlaufenden Querschnittsebene angeordnet ist, so laufen die Stützringe kreisförmig bzw. gerade und nicht schräg bzw. elliptisch um die Stützprothesenhauptachse um. Hierdurch werden zusätzliche Kraftkomponenten, die bei einer schrägen Anordnung der Stützringe auftreten würden, vermieden.

Des Weiteren kann die Mäanderform des Filaments eines Stützringes mindestens zwei unterschiedliche Krümmungsbogenformen bzw. -größen aufweisen, die gemeinsam ein wiederkehrendes Muster der Mäanderform bilden. Hier könnte man auch von einer periodisch umlaufenden Musterfolge sprechen. Während bei den üblichen Gefäßstents gleichmäßige Mäandermuster eingesetzt werden, wird beim erfindungsgemäßen Gefäßstent ein unregelmäßiges Muster aufgrund unterschiedlicher Krümmungsbogenformen verwendet, so dass die Periode größer ist. Hierdurch lässt sich innerhalb eines bestimmten Bereichs ein größerer Einfluss auf das Verformungsverhalten beim Aufdehnen nehmen. Insbesondere wenn engere und breitere Krümmungsbögen sowie längere und kürzere Krümmungsbögen verwendet werden, lässt sich ein gezieltes stufenweises Expandieren des Gefäßstents durchführen.

Des Weiteren kann vorteilhafterweise ein zwischen zwei miteinander verbundenen Überlappungsbereichen gebildetes Prothesefenster eine erste, von dem ersten Stützring gebildete Rahmenhälfte und eine zweite, von dem benachbarten zweiten Stützring gebildete Rahmenhälfte aufweisen, wobei beide Rahmenhälften punktsymmetrisch zueinander ausgestaltet sind. Sofern eine unregelmäßige Mäanderform gewählt wird, führt eine Punktsymmetrie der Fensterrahmenhälfte zu den gleichen Expansionseigenschaften jedoch in die entgegengesetzte Richtung, wodurch sich eine Vergleichmäßigung der Aufdehneigenschaften ergibt.

In diesem Zusammenhang hat sich hinsichtlich einer reduzierten Längenveränderung eine Ausführungsform als vorteilhaft herausgestellt, bei der der Winkel α bei mindestens einem Überlappungsbereich 15° - 25° und bei den anderen Überlappungsbereichen 165° bis 155° oder bei mindestens einem Überlappungsbereich 20° - 30° und bei einem anderen Überlappungsbereich 150° - 160° beträgt. Beide Winkel sind von der gleichen Bezugslinie aus gemessen, weshalb von einer gegengerichteten Neigung der Überlappungsbereich gesprochen werden kann, so dass zum Teil sich die Überlappungsbereiche beim Aufdehnen im Uhrzeigersinn und zum Teil entgegen dem Uhrzeigersinn aufrichten, was wiederum zu einer Vergleichmäßigung der Expansion führt.

Eine Vergleichmäßigung der Spannungssituation lässt sich insbesondere bei einer Variante dadurch erzielen, dass ein Stützring umlaufend abwechselnd auf der einen Seite mit einem benachbarten Stützring und auf der anderen Seite mit einem weiteren benachbarten Stützring verbunden ist. Demnach ist ein Stützring zwischen zwei in Umfangsrichtung nacheinander angeordneten Überlappungsbereichen auf der anderen Seite mit einem weiteren Stützring verbunden. Die Verbindungsstellen bzw. Überlappungsbereiche wechseln somit zickzackförmig die Seite entlang des Umfangs eines Stützrings.

Die Verzahnung der Stützringe ineinander kann günstigerweise so gewählt werden, dass das Verhältnis von der Länge des Überlappungsbereichs, in Längsrichtung der Stützprothese gesehen, zur Breite des Stützrings größer ist als 0,1, bevorzugt größer als 0,2. Hierdurch ist in aller Regel sichergestellt, dass nicht nur die Scheitelbereiche der Krümmungsbögen sich überlappen, sondern auch Teile der Schenkel der Krümmungsbögen den Überlappungsbereich mitbilden. Diese Ausgestaltung hat positiven Einfluss auf das Verformungsverhalten und wirkt insbesondere einem Aufstellen der Krümmungsbögen beim Expansionsvorgang entgegen.

Des Weiteren kann jeder Krümmungsbogen einen ersten Schenkel, einen zweiten Schenkel und eine sich zwischen diesen Schenkeln erstreckenden Scheitelabschnitt umfassen, wobei der erste und zweite Schenkel in die gleiche Richtung gekrümmt ausgestaltet sind. Eine derartige zusätzliche Krümmung, so dass die Krümmungsbögen den Umriss einer Flosse oder Krallenform ausformen, ist bislang unüblich. Auch diese Konstruktionsmaßnahme dient hauptsächlich einer Vergleichmäßigung des Aufdehnverhaltens des Gefäßstents. Eine diesbezügliche zusätzliche Ausgestaltung sieht vor, dass die ersten und zweiten Schenkel der Krümmungsbögen eines Stützrings in die gleiche Richtung gekrümmt sind und die ersten und zweiten Schenkel der Krümmungsbögen der angrenzenden Stützringe in die entgegengesetzte Richtung gekrümmt sind. Hierdurch ergibt sich wieder eine Vergleichmäßigung des Aufdehnverhaltens, da die durch diese Form erzielte Wirkung sich in beide Richtungen entfaltet. Darüber hinaus führt dies Maßnahme dazu, dass ein möglichst langer Überlappungsbereich ausgeformt werden kann, ohne dass der Platzaufwand zu groß wird.

Mittels einer weiteren Ausgestaltung kann auf dem Filament eine Beschichtung vorgesehen sein, die Siliziumdioxid aufweist. Demnach kann also eine glasähnliche Beschichtung vorgesehen sein. Das Siliziumdioxid kann in amorpher oder kristalliner oder halbkristalliner Form vorliegen.

Die Eigenschaften der Beschichtung können weiterhin durch zumindest eine Beimischung, die in den Beschichtung enthalten ist, modifiziert werden, wobei die Beimischung ausgewählt sein kann aus Aluminiumoxid, Titanoxid, Kalziumverbindungen, Natriumoxid, gemahlenem Oxid, Magnesiumoxid, Selenoxid und Hydrooxiden, insbesondere Hydrooxiden der vorgenannten Metalle. Besonders bevorzugte Beimischungen sind Aluminiumoxid und Titanoxid.

Wenn eine Beimischung von Siliziumdioxid verwendet wird, kann der Anteil der Beimischung an der Gesamtmenge der Beschichtung vorzugsweise 0,5 bis 50 Gew.-% betragen.

Um über die gesamte Oberfläche der Stützprothese die gewünschte Oberflächeneigenschaft zu bewahren, wird es bevorzugt, dass die Beschichtung im wesentlichen porenfrei ist.

Bei bestimmten Ausführungsformen kann es jedoch ebenfalls bevorzugt werden, dass die Beschichtung Poren für eine Funktionalisierung mit weiteren Substanzen, die nach der eigentlichen Beschichtung auf die Beschichtung aufgebracht werden und sich in de Poren ablagern, aufweist. Dementsprechend kann die Beschichtung einen zusätzlichen, auch nur partiell oder punktuell vorhandenen Funktionalisierungsauftrag aufweisen. Ein solcher Auftrag kann der medizinischen Zweckbestimmung der Stützprothese entsprechen und einer Beeinflussung des Wachstums von umgebenden Gewebe, einer Abtötung unerwünschten Gewebes, den Aufbau einer Beziehung zwischen der Stützprothese und Gewebe etc. beinhalten. Der Funktionalisierungsauftrag kann beispielsweise zumindest ein Medikament und/oder zumindest ein Zellgift enthalten.

Ein großer Vorteil der erfindungsgemäßen Stützprothese ist darin zu sehen, dass die Beschichtung extrem dünn aufgebracht werden kann, nämlich vorzugsweise im Nano-Bereich, also im Bereich einiger Atomschichten, was es gestattet, bei der Herstellung der Stützprothese im wesentlichen die Endabmessungen einstellen zu können, ohne auf möglicherweise nicht exakt vorhersehbare Dimensionierungsänderungen durch die Beschichtung Rücksicht nehmen zu müssen. Vorzugsweise beträgt die Dicke der erfindungsgemäßen Beschichtung 0,1 bis 1000 nm. Es versteht sich jedoch, dass sowohl dünnere als auch dickere Beschichtungen möglich sind. Maßgebend bei der Wahl der Schichtdicke ist die Anforderung, dass bei Expansion der Stützprothese im Körper die Beschichtung nicht beschädigt wird und keine zusätzlichen Poren entstehen.

Die Beschichtung kann in einem einzelnen Schritt aufgetragen werden, und somit eine einlagige Schicht bilden, kann jedoch in einer bevorzugten Ausführungsform auch aus mehreren, sukzessive aufgebrachten Schichten bestehen. Bei mehrlagigem Aufbringen kann die Zusammensetzung jeder einzelnen Schicht individuell festgelegt werden. Die Stützprothese umfasst ein die Grundstruktur bildenden Träger, der zumindest teilweise mit der Beschichtung versehen ist.

Der Träger ist vorzugsweise aus einem schwer abbaubaren Material aufgebaut, wobei unter "schwer abbaubar" eine Eigenschaft zu verstehen ist, bei der das Material nach Implantation in einen Körper für zumindest ein Jahr keine sichtbaren Abbauerscheinungen zeigt.

Der Träger der Stützprothese kann übliche Materialien, wie Karbon, PTFE, Dakron, Metall-Legierungen oder PHA umfassen, wobei insbesondere Stahllegierungen bevorzugte Materialien sind.

Bevorzugt werden die für den Träger verwendbaren Metall-Legierungen aus der Gruppe der Edelstähle ausgewählt.

Ein weiteres bevorzugtes Material für den Träger ist ein Formgedächtnismetall, insbesondere Nickel-Titan-Legierungen, die aufgrund ihrer Fähigkeiten zur eigenständige Formveränderung bei derartigen Stützprothesen Einsatz finden.

Ein Verfahren zur Herstellung der Stützprothese kann folgende Schritte umfassen:
- Bereitstellen eines die Grundstruktur bildenden Trägers; und
- Aufbringen einer Siliziumdioxid enthaltenden Beschichtung mittels eines Plasmabeschichtungsverfahren.

Um die bei bestimmten Ausführungsformen gewünschten Poren zur Aufnahme von Funktionalisierungsmitteln zu erhalten, wird es weiterhin bevorzugt, dass das Verfahren einen Schritt des Erzeugens von Poren in der Beschichtung mittels eines Neutronenbeschusses umfasst. Hierfür können Neutronenquellen wie beispielsweise Teilchenbeschleuniger verwendet werden. Eine weitere Variante zum Erzeugen von Funktionsporen besteht darin, die Poren mittels Laserlicht herzustellen.

Die oben beschriebene Beschichtung verhindert aufgrund ihrer inerten, glasartigen Oberfläche mit Siliziumdioxid einen Bewuchs mit Zellen des Körpers bzw. einer Anhaftung solcher Zellen weitgehend, da sie aufgrund ihrer Härte einer Beschädigung beim Einbringen der Stützprothese in den Körper entgegenwirkt und damit die Handhabung vereinfacht, die aufgrund der Dünne der Beschichtung eine einfache Gestaltung der Stützprothese gestattet. Des Weiteren weist sie eine verminderte Reibung durch geringere Rauhigkeitswerte und damit eine kleinere Belastung für Blutkomponenten und niedrigere Koagelbildung auf, wobei es auch nach längerer Verbleibdauer keinerlei Abbau der Beschichtung gibt.

Bei einer weiteren Ausgestaltung ist vorgesehen, dass jeder Krümmungsbogen eine Stelle kleinsten Querschnitts aufweist und sich das Filament zumindest abschnittsweise zu der Stelle kleinsten Querschnitts hin kontinuierlich verjüngt. Aufgrund einer Zugbelastung im Mäandermuster setzt die Verformung der Krümmungsbögen an den jeweiligen Stellen gleichen Querschnitts nahezu zeitgleich ein und setzt sich dann kontinuierlich in die anderen Bereiche der Krümmungsbögen fort. Insbesondere wird durch die kontinuierliche Verjüngung der Krümmungsbögen zur Stelle geringsten Querschnitts hin verhindert, dass die Streckbewegung des Mäandermusters zunächst zur Streckung einzelner Krümmungsbögen führt. Grund hierfür ist, dass vor einer vollständigen Streckung eines Krümmungsbogens die Verformung eines benachbarten Krümmungsbogens an der Stelle kleinsten Querschnitts einsetzt. Hierdurch wird eine gleichmäßige Aufdehnung der Stützprothese ermöglicht.

Des Weiteren kann eine Stelle kleinsten Querschnitts eines Krümmungsbogens abseits einer Mittellinie des Krümmungsbogens angeordnet sein. Die Stelle kleinsten Querschnitts kann sich dabei im Bereich des Scheitels des Krümmungsbogens befinden.

Der Vollständigkeit halber sei noch erwähnt, dass bei der Verwendung von einer Vielzahl nebeneinander angeordneter Stützringe, die beiden jeweils außenliegenden Stützringe zumindest nach außen gerichtete Krümmungsbögen aufweisen können, die wieder vergleichmäßigt sind, so dass die Scheitelpunkte dieser Krümmungsbögen in einer Ebene liegen und keiner der Krümmungsbögen nach außen übersteht.

Im Folgenden wird nunmehr die Erfindung anhand einer Zeichnung näher erläutert. Es zeigen:
- Fig. 1: eine schematische Darstellung einer Stützprothese mit vier Stützringen,
- Fig. 2: eine schematische Darstellung einer Abwicklung einer ersten Ausführungsform einer Stützprothese,
- Fig. 3: eine schematische Ansicht einer Abwicklung einer zweiten Ausführungsform einer Stützprothese,
- Fig. 4: eine schematische Ansicht einer Abwicklung einer dritten Ausführungsform einer Stützprothese,
- Fig.5: eine maßstabsgetreue Ansicht einer Abwicklung einer vierten Ausführungsform einer Stützprothese,
- Fig. 6: eine vergrößerte Darstellung eines Scheitelbereichs eines Krümmungsbogens gemäß einer weiteren Variante,

- Fig. 7: einen Querschnitt des Krümmungsbogens aus Fig. 6 entlang der Schnittlinie VII-VII,
- Fig. 8: einen Querschnitt durch den Krümmungsbogen aus Fig. 6 entlang der Schnittlinie VIII-VIII,
- Fig. 9: einen Querschnitt durch den Krümmungsbogen aus Fig. 6 entlang der Schnittlinie IX-IX, und
- Fig. 10: den Querschnitt ähnlich Fig. 9 in einer Variante mit Beschichtung.

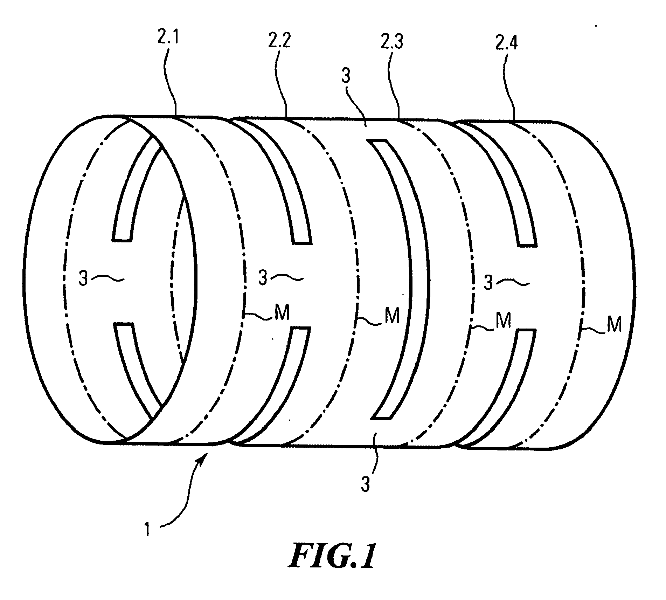

Anhand der Fig. 1 wird im Folgenden der prinzipielle Aufbau anhand einer schematischen Darstellung erläutert. Aus Vereinfachungsgründen sind feine Strukturen vollflächig dargestellt und die einzelnen Mäanderformen bzw. Filamente nicht zu erkennen.

Die in Fig. 1 rohrförmig dargestellte Stützprothese 1 umfasst vier nebeneinander liegende Stützringe 2.1, 2.2, 2.3. und 2.4, die über zwischen den Stützringen 2.1 - 2.4 angeordneten Verbindungsstellen 3 miteinander verbunden sind. Bei dem vorliegenden Beispiel sind jeweils zwei Verbindungsstellen zwischen den einzelnen Stützringen 2.1 - 2.4 angeordnet.

Die Stützprothese 1 ist mittels eines Ballonkatheters aus der in Fig. 1 dargestellten Stellung in eine aufgedehnte Stellung expandierbar. Hierzu weist jeder Stützring 2.1 - 2.4 ein in Fig. 1 nicht dargestelltes Filament auf, das mäanderförmig umläuft. Diese Mäanderform verleiht jedem Stützring 2.1 - 2.4 die entsprechende Expansionseigenschaften. Die Verbindungsstellen 3 werden, wie im Folgenden noch näher beschrieben wird, durch Überlappungen von Krümmungsbögen benachbarter Stützringe 2.1 - 2.4 gebildet. In Fig. 1 sind auch die umlaufenden Mittellinien M der Stützringe 2.1 - 2.4 eingezeichnet.

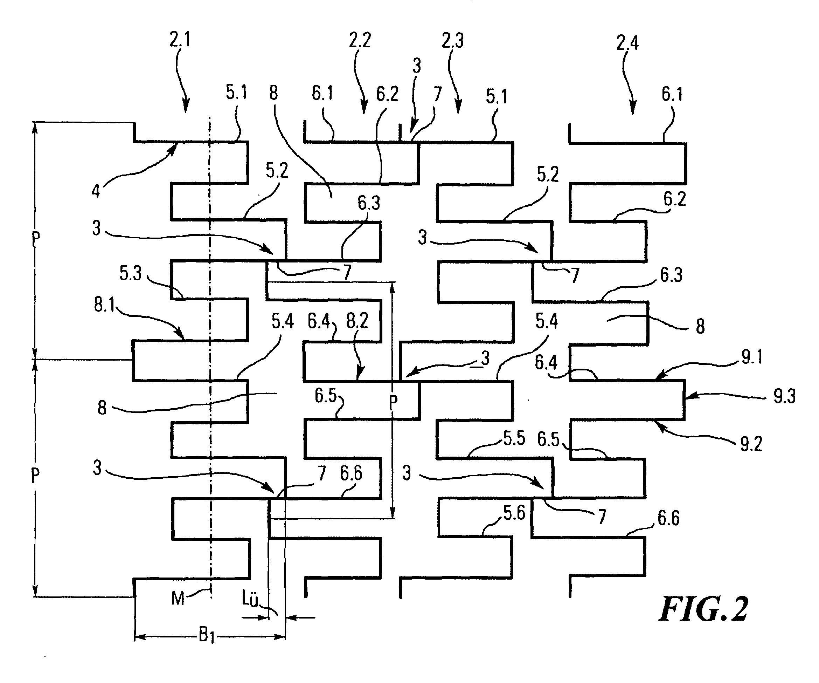

Anhand der Fig. 2 bis 4 werden nun im Folgenden schematische Abwicklungsbeispiele der Mäanderformen der in Fig. 1 schematisch dargestellten Stützprothese näher erläutert. Wie in Fig. 1 sind in Fig. 2 vier Stützringe 2.1 - 2.4 (in der Abwicklung als flachliegende Bänder dargestellt) nebeneinander angeordnet. Die Abwicklung zeigt den gesamten Umfang der Stützprothese 1.

Jeder Stützring 2.1 - 2.4 besteht aus einem mäanderförmig umlaufenden Filament 4, das in der Fig. 2 aus Vereinfachungsgründen als Strich dargestellt, in der konkreten Anwendung jedoch ein dreidimensionales dünnes und schmales Band darstellt. Im vorliegenden Beispiel weist der Stützring 2.1 sechs in Richtung des zweiten Stützrings 2.2 gerichtete Krümmungsbögen 5.1 - 5.6 auf. Während die Krümmungsbögen 5.1, 5.3, 5.4 und 5.6 in gleicher Höhe enden, stehen die Krümmungsbögen 5.2 und 5.5 über diese über. Der Stützring 2.2 weist sechs in Richtung des ersten Stützrings 2.1 gerichtete Krümmungsbögen 6.1 - 6.6 auf. Während die Krümmungsbögen 6.1, 6.2, 6.4 und 6.5 auf gleicher Höhe und im Abstand zum ersten Stützring 2.1 enden, stehen die Krümmungsbögen 6.3 und 6.6 auf dieser Seite über und überlappen die zugeordneten Krümmungsbögen 5.2 und 5.5 des ersten Stützrings 2.1 entsprechend. Die Überlappung erfolgt derart, dass gleichzeitig eine Verbindung im Überlappungsbereich stattfindet, so dass die Verbindungsstelle 3 gebildet ist. Ein kleiner Filamentabschnitt 7 ist beiden Krümmungsbögen 5.2 und 6.3 bzw. 5.5 und 6.6 gemeinsam zugeordnet. Der Querschnitt des gemeinsamen Filamentabschnitts 7 ist gegenüber dem restlichen Querschnitt des Krümmungsbogens im Wesentlichen nicht verändert, um Materialanhäufungen soweit wie möglich zu vermeiden.

Die Breite B₁ des Stützrings 2.1 beträgt im vorliegenden Beispiel ca. 2,5 mm und die Länge L_{Ü} des Überlappungsbereichs bzw. gemeinsamen Filamentabschnitts 7 0,3125 mm. Daher beträgt das Verhältnis V zwischen L_{Ü} und B₁ im vorliegenden Fall 0,125.

Die Krümmungsbögen 5.1, 5.2 und 5.3 bilden ein sich periodisch wiederholendes Muster mit der Periode P. Im vorliegenden Fall kommt dieses Muster zweimal am Umfang der Stützprothese 1 vor, so dass die Krümmungsbögen 5.4, 5.5 und 5.6 die gleiche Mäanderform und -größe darstellen. Der Stützring 2.2 ist im wesentlichen gleich zum Stützring 2.1 ausgestaltet, jedoch phasenverschoben angeordnet. Der Stützring 2.3 ist wieder phasengleich mit dem Stützring 2.1 angeordnet und identisch ausgestaltet. Der Stützring 2.4 ist phasengleich und identisch zum Stützring 2.2 ausgestaltet.

Zwischen den beiden Verbindungsstellen 3 der Stützringe 2.1 und 2.2 ist jeweils ein Prothesenfenster 8 gebildet, das eine im wesentlichen von dem ersten Stützring 2.1 gebildete Rahmenhälfte 8.1 und eine im wesentlichen von dem zweiten Stützring 2.2 gebildete Rahmenhälfte 8.2 aufweist. Die Rahmenhälften 8.1 und 8.2 sind punktsymmetrisch zueinander ausgestaltet. Jeweils zwei dieser Prothesenfenster 8 sind zwischen dem ersten und zweiten Stützring 2.1 und 2.2 angeordnet. Gleiches gilt für entsprechende Prothesenfenster 8 zwischen dem zweiten und dritten Stützring 2.2 und 2.3 und dem dritten und vierten Stützring 2.3 und 2.4.

Beispielhaft wird anhand des Stützrings 2.4 beschrieben, dass jeder Krümmungsbogen einen ersten Schenkel 9.1, einen zweiten Schenkel 9.2 und einen diese miteinander verbindenden Scheitelbereich 9.3 umfasst. Im vorliegenden Beispiel weisen alle Krümmungsbögen eine gleiche Breite auf und die Schenkel 9.1 und 9.2 verlaufen im wesentlichen achsparallel zu einer Prothesenhauptachse A. Somit verläuft auch der gemeinsame Filamentabschnitt 7 im wesentlichen achsparallel. Aus Vereinfachungsgründen ist in dem vorliegenden Beispiel der Scheitelbereich 9.3 gerade dargestellt; dieser wird jedoch in aller Regel durch einen Übergansbogen gebildet. Aus der Fig. 2 ist sehr schön zu erkennen, dass im vorliegenden Beispiel alle Krümmungsbögen die gleiche Breite aufweisen. Nachdem es sich bei der Fig. 2 um eine schematische Darstellung handelt, an der das Grundprinzip klargemacht werden soll, soll verstanden sein, dass in der Praxis in aller Regel keine scharfen Übergänge zwischen einzelnen Krümmungsbögenabschnitten ausgestaltet werden, sondern Übergänge in der Form von Radien oder Bogenabschnitten verwendet werden. Wird die in Fig. 2 dargestellte Struktur in eine Rohrform gewickelt oder aus einem Rohr herausgearbeitet, so kann diese in nachfolgenden Schritten aufgedehnt werden. Nachdem die gemeinsamen Filamentabschnitte 7 ein etwas anderes Verformungsverhalten aufweisen, als die restlichen Krümmungsbögen, verschwenken sich diese beim Aufdehnen derart, dass die Stützringe 2.1 - 2.4 in axialer Richtung auseinander geschoben werden, während sich diese im Umfang vergrößern. Dadurch wird die Länge im wesentlichen beibehalten. Gewünscht ist eine maximale Längenänderung von 5%.

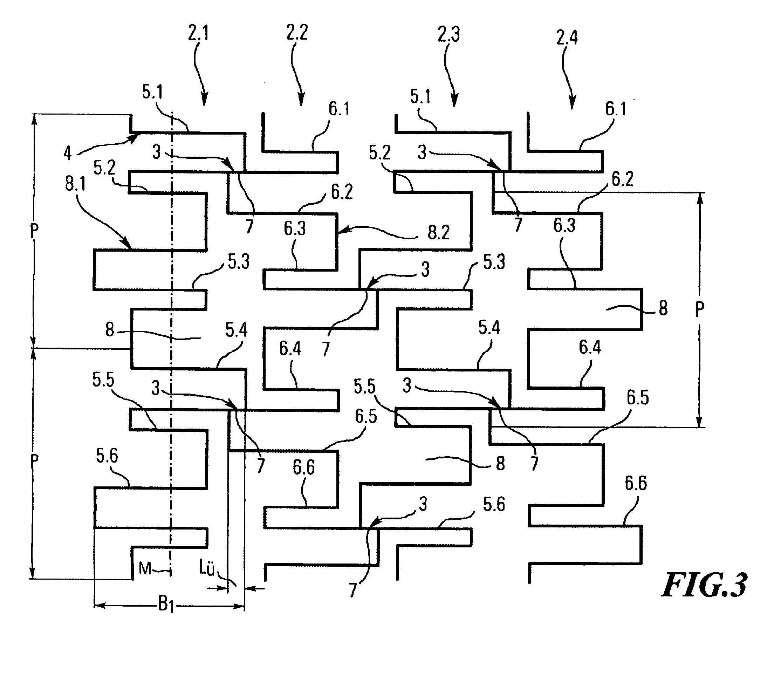

Im Folgenden wird anhand er Fig. 3 ein weiteres Ausführungsbeispiel näher erläutert. Es wird im Folgenden nur auf die wesentlichen Unterschiede eingegangen. Soweit baugleiche bzw. funktionsgleiche Elemente verwendet werden, werden die gleichen Bezugsziffem benutzt. Ansonsten wird auf die obige Beschreibung verwiesen.

Der Hauptunterschied besteht darin, dass die Mäanderform innerhalb einer Periode drei unterschiedliche Krümmungsbogenformen aufweist. Die Krümmungsbögen 5.1, 5.2 und 5.3 haben jeweils eine andere Breite, so dass sich eine etwas unregelmäßigere Struktur ergibt. Sämtliche anderen oben beschriebenen Parameter bleiben im wesentlichen gleich. Z.B. sind die Prothesenfenster 8 weiterhin hinsichtlich ihrer beiden Rahmenhälften 8.1 und 8.2 punktsymmetrisch ausgestaltet, die Stützringe 2.1 und 2.3 sind phasengleich und identisch ausgestaltet, die Stützringe 2.2 und 2.4 sind phasengleich und identisch ausgestaltet etc.

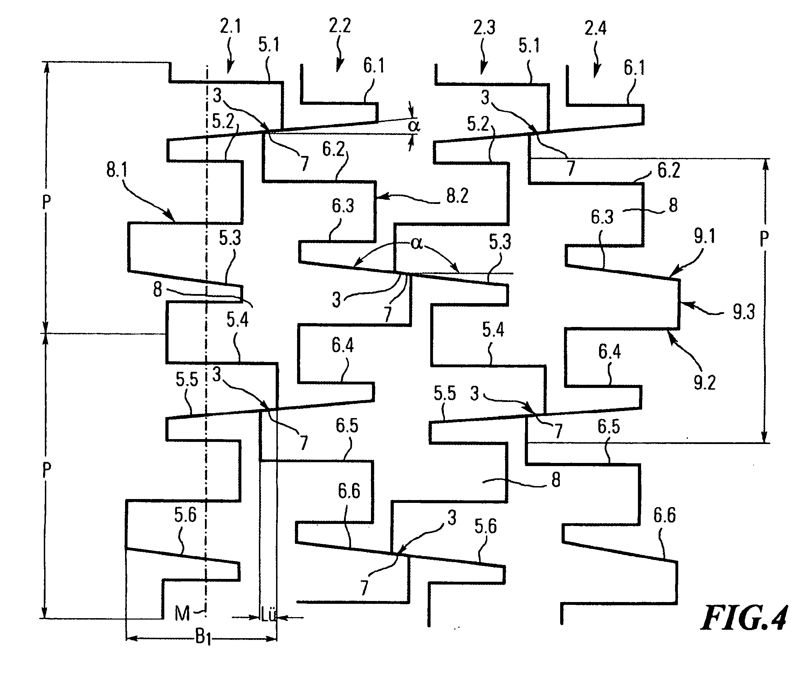

Auch hinsichtlich der Ausführungsform gemäß der Fig. 4 wird nur auf die wesentlichen Unterschiede zu den vorangegangenen Ausführungsbeispielen eingegangen. Deshalb werden auch hier wieder für gleiche und funktionsgleiche Bauelemente gleiche Bezugsziffern verwendet und auf die obige Beschreibung verwiesen.

Im Gegensatz zu der Ausführungsform der Fig. 3 sind neben der unterschiedlichen Breiten der drei Kürmmungsbögen 5.1, 5.2 und 5.3 in einer Phase noch zusätzlich schräg verlaufende Schenkel 9.1 und 9.2 von einigen Krümmungsbögen (z.B. 5.1 und 6.2) vorgesehen. Hierdurch ist der gemeinsame Filamentabschnitt 7 im Übergangsbereich schräg ausgerichtet, und zwar in einem Winkel α, der im vorliegenden Beispiel zwischen dem ersten und zweiten Stützring 2.1 und 2.2 ca. 5° und zwischen den Stützringen 2.2 und 2.3 ca. 175° beträgt. Gemessen wird relativ zu einer Bezugslinie, die eine Parallele zur Prothesenhauptachse A darstellt. Die Ausrichtung der gemeinsamen Filamentabschnitte 7 zwischen dem ersten und zweiten Stützring 2.1 und 2.2 ist gegengerichtet zur Ausrichtung der gemeinsamen Filamentabschnitte 7 zwischen dem zweiten Stützring 2.2 und dritten Stützring 2.3. Darüber hinaus sind wie bei den vorangegangenen Ausführungsbeispielen die Verbindungsstellen 3 zwischen dem zweiten Stützring 2.2 und dem dritten Stützring 2.3 zwischen den Verbindungsstellen 3 des ersten Stützrings 2.1 und dem zweiten Stützring 2.2 angeordnet. Durch diese Ausgestaltung verschwenken die gemeinsamen Filamentabschnitte 7 beim Aufdehnen der Stützprothese 1 in entgegengesetzter Richtung. Des Weiteren sind diese einfacher zu Verschwenken als die achseparallele Ausrichtung gemäß der Fig. 2 und 3. Hierdurch wird noch einmal eine Vergleichmäßigung erzielt. Auch durch die unterschiedlichen Breiten der Krümmungsbögen ergibt sich ein dosiertes, eventuell auch stufenweises Aufdehnen.

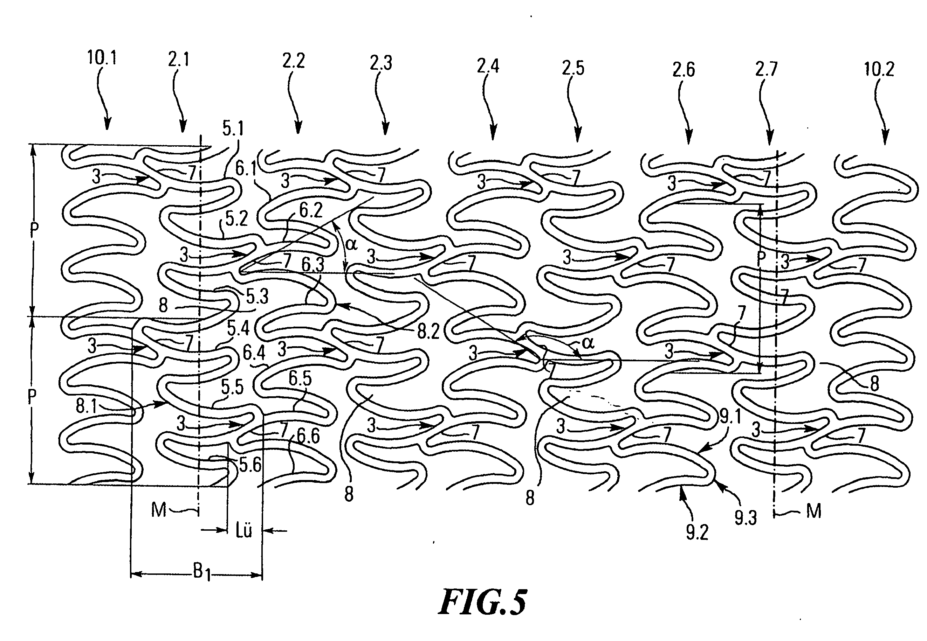

Anhand der Fig. 5 wird nunmehr die zur Zeit favorisierte Ausgestaltung näher erläutert. Auch hier wird soweit wie möglich auf die obige Beschreibung zurückgegriffen und für gleichartige und funktionsgleichartige Bauelemente die gleichen Bezugsziffern verwendet.

Damit die Enden der Stützprothese 1 möglichst gleichmäßig ausgeformt sind, sind jeweils ein Endstützring 10.1 und 10.2 vorgesehen. Bei diesen Endstützringen enden sämtliche Krümmungsbögen, die zur Stirnseite hin vorstehen auf gleicher Höhe. Insofern erfüllt der Endstützring 10.1 nicht alle Symmetriekriterien, wie in den vorangegangenen Beispielen. Insgesamt sind sieben Stützringe 2.1 - 2.7 vorhanden. Auch der Endstützring 10.2 erfüllt nicht sämtliche Symmetriekriterien in Bezug auf den letzten Stützring 2.7. Die Stützringe 2.1, 2.3, 2.5 und 2.7 sind phasengleich und identisch ausgestaltet. Gleiches gilt für die Stützringe 2.2, 2.4 und 2.6, die ebenfalls phasengleich und identisch zueinander ausgestaltet sind. Sowohl die Schenkel 9.1 und 9.2 als auch die Scheitelbereiche 9.3 sind bei dem vorliegenden Beispiel gekrümmt ausgestaltet. Bei dem Stützring 2.1 erfolgt die Krümmung der Schenkel 9.1 und 9.3 derart, dass die konvexen Seiten in der Fig. 5 nach unten weisen und die konkaven Seiten nach oben. Demnach sind sämtliche Krürmmungsbögen 5.1 - 5.6 in die gleiche Richtung gekrümmt und stellen in ihrer Kontur im wesentlichen eine Krallen- bzw. Flossenform dar. Das Verhältnis V von L_{Ü} und B₁ beträgt im vorliegenden Fall 0,265, wobei B₁ ca. 2,54 mm und L_{Ü} ca. 0,69 mm beträgt.

Der zweite Stützring 2.2 ist so ausgestaltet, dass die Schenkel 9.1 und 9.2 der Krümmungsbögen 6.1 - 6.6 mit ihrer konvexen Seite in der Fig. 5 nach oben und mit ihrer konkaven Seite nach unten weisen und somit entgegengesetzt gekrümmt sind zu den Krümmungsbögen 5.1 - 5.6 des Stützrings 2.1. Hierdurch ergibt sich wieder eine Punktsymmetrie der Prothesenfenster 8. Insgesamt sind demnach drei verschiedene Krümmungsbögen (z.B. 5.1, 5.2 und 5.3) in jeweils einem der Stützringe 2.1 - 2.7 vorhanden.

Der Winkel α beträgt zwischen den Stützringen 2.1 und 2.2, sowie 2.3 und 2.4, 2.5 und 2.6 jeweils 25° bzw. zwischen den Stützringen 2.2 und 2.3, sowie 2.4 und 2.5 und 2.6 und 2.7 jeweils 155°. Die Verbindungsstellen 3 zwischen dem ersten Stützring 2.1 und dem Endstützring 10.1 sowie dem letzten Stützring 2.7 und dem Endstützring 10.2 sind in gleicher Weise ausgestaltet.

Beim Aufdehnen der Stützprothese 1 schwenken demnach die gemeinsamen Filament-abschnitte 7 zwischen dem ersten und zweiten Stützring 2.1 und 2.2 entgegen dem Uhrzeigersinn und zwischen den Stützringen 2.2 und 2.3 mit dem Uhrzeigersinn. Entsprechendes gilt für die Verbindungsstellen 3 zwischen den jeweils anderen Stützringen in Abhängigkeit der Ausrichtung der gemeinsamen Filamentabschnitte 7. Die Längenänderung beim Aufdehnen beträgt daher maximal 2 bis 3 %. Hierfür sorgen die Verbindungsstellen 3 aufgrund ihrer Ausrichtung und Ausformung. Darüber hinaus sorgen die gebogenen bzw. nochmals gekrümmten Krümmungsbögen (5.1 - 5.6, 61. - 6.6) dafür, dass eine noch geringere Neigung zum Aufstellen der Krümmungsbögen (5.1 - 5.6, 6.1 - 6.6) beim Aufdehnen vorhanden ist. Darüber hinaus führt die Form der Prothesenfenster 8 dazu, dass diese sich nicht zu weit beim Aufdehnen öffnen und somit ein gleichmäßiges Aufdehnverhalten haben. Beim anfänglichen Durchmesser der Stützprothese 1 von 2 mm beträgt die Fläche eines Prothesenfensters 8 ca. 4,89 mm². Beim anschließenden Aufdehnen auf einen Durchmesser von 4 mm beträgt die Fläche ca. 11,26 mm², was einem Vergrößerungsfaktor von 2,3 entspricht. Derartige rohrförmige Stützprothesen 1 werden vor dem Implantieren auf einen Ballonkatheter aufgecrimpt, wodurch sich deren Durchmesser nochmals verringert, z.B. auf 1 mm. Die Fläche der Prothesenfenster 8 beträgt dann nur noch 2,81 mm², was einer Verringerung von fast auf die Hälfte entspricht.

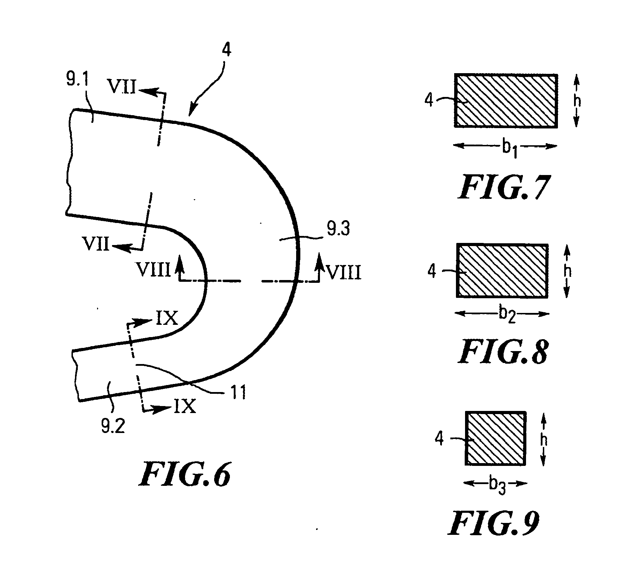

Im Folgenden wird anhand der Fig. 6 bis 9 eine Ausgestaltung in der Feinstruktur der Krümmungsbögen näher erläutert. Derartige Maßnahmen in der Feinstruktur können auch bei sämtlichen vorangegangenen Ausführungsbeispielen Anwendung finden. Die bezüglich der oben genannten Ausführungsbeispiele erwähnten Symmetriekriterien sollen aber auch unabhängig (also mit oder ohne) solcher evtl. vorhandener zusätzlicher Maßnahmen verstanden sein und bleiben gegebenenfalls bei der Symmetriebetrachtung unberücksichtigt.

In Fig. 2 ist eine vergrößerte Darstellung eines Krümmungsbogens (z.B. 5.1) gezeigt. Der Krümmungsbogen weist eine Stelle kleinsten Querschnitts 11 auf. Während der Schenkel 9.1 und der Schenkel 9.2 jeweils einen gleichbleibenden Querschnitt aufweisen, verjüngt sich der Querschnitt im Bereich des Scheitelbereichs 9.3 kontinuierlich. Erzeugt wird dies durch einen äußeren und inneren Krümmungsradius, die einen versetzten Mittelpunkt zueinander haben.

Aufgrund der Tatsache, dass die Stützprothese 1 aus einem Metallröhrchen gefertigt wird, werden die Stützringe 2.1 - 2.7 sowie 10.1 und 10.2 mit Hilfe eines Lasers aus dem Metallröhrchen herausgearbeitet. Deshalb weisen die in den Fig. 7 bis 9 dargestellten Querschnitte jeweils die gleiche Höhe h auf. Die Querschnittsflächen unterscheiden sich jedoch hinsichtlich der Breite. Diese nimmt von b₁ bis b₃ kontinuierlich ab.

Die Art und Weise der Ausgestaltung der Stelle kleinsten Querschnitts 11 sowie deren Anordnung kann von diesem Beispiel abweichen. Diese könnte auch symmetrisch in der Mitte des Scheitelbereichs 9.3 angeordnet sein, wobei die beiden Schenkel 9.1, 9.2 den gleichen Querschnitt aufweisen. Dies hängt maßgeblich von den gewünschten Verformungseigenschaften, insbesondere um welchen Punkt sich die Struktur aufbiegen soll, ab. Auch die Scheitelbereiche 9.3, die an die gemeinsamen Filamentabschnitte 7 angrenzen bzw. einen Teil von diesen bilden, können derart ausgestaltet sein. Die Stellen kleinsten Querschnitts 11 können sich auch über einen längeren Abschnitt der Krümmungsbögen erstrecken.

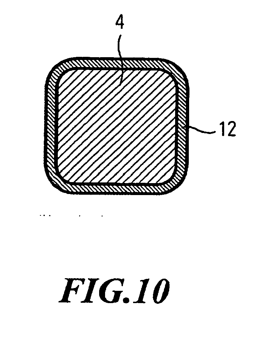

Eine weitere Ausgestaltung zeigt Fig. 10, bei der eine Beschichtung 12 vorgesehen ist. Bei der Beschichtung 12 handelt es sich um eine Siliziumdioxidhaltige Beschichtung, also einer glasähnlichen Beschichtung. Das Trägermaterial ist bevorzugt eine Metalllegierung, insbesondere Stahllegierung, auf der diese Beschichtung 12 aufgebracht ist. Eine solche Beschichtung 12 soll insbesondere unkontrollierte Ablagerungen wie Plaque etc. verhindern. Insbesondere wird aufgrund der glasartigen Oberfläche mit Siliziumdioxid ein Bewuchs mit Zellen des Körpers bzw. eine Anhaftung solcher Zellen weitgehend verhindert, die aufgrund ihrer Härte eine Beschädigung beim Anbringen der Stützprothese 1 in den Körper entgegenwirkt und damit die Handhabung vereinfacht. Aufgrund der Dünne der Beschichtung ist eine einfache Gestaltung der Stützprothese 1 gestattet. Des Weiteren weist diese eine verminderte Reibung durch geringere Rauhigkeitswerte und damit eine kleinere Belastung für Blutkomponenten und niedriger Koagelbildung auf. Auch nach längerer Verbleibdauer im Körper gibt es keinerlei Abbau der Beschichtung. Im Zusammenspiel mit der erfindungsgemäßen Ausgestaltung der Stützringe ist dafür gesorgt, dass beim Aufdehnvorgang die Beschichtung 12 keinen Schaden nimmt, insbesondere stellenweise abplatzt. Aufgrund der gewählten Struktur werden insbesondere Torsionskräfte beim Aufdehnvorgang gering gehalten. In Fig. 10 ist sehr gut zu erkennen, dass die Kanten des Filaments 4 durch Elektropolieren abgerundet sind.

## Patentansprüche

1. Rohrförmige Stützprothese (1) für Gefäße oder interkorporale Lumina mit mindestens zwei, entlang einer Stützprothesenachse (A) nebeneinander angeordneten und an mindestens einer Verbindungsstelle (3) miteinander verbundenen expandierbaren Stützringen (2.1 - 2.7, 10.1, 10.2), die jeweils von einem mäanderförmig zu mehreren Krümmungsbögen (5.1 - 5.6, 6.1 - 6.6) gewundenen Filament (4) gebildet sind, wobei sich mindestens ein Krümmungsbogen (6.1) eines ersten Stützrings (2.2) und ein Krümmungsbogen (5.1) eines benachbarteni zweiten Stützrings (2.3) in Umfangsrichtung seitlich überlappen und sich mindestens ein Krümmungsbogen (6.3) des ersten Stützrings (2.2) und ein Krümmungsbogen (5.2) eines benachbarten dritten Stützrings (2.1) in Umfangsrichtung seitlich überlappen, und im Überlappungsbereich jeweils die Verbindungsstelle (3) gebildet ist, jeder Krümmungsbogen (5.1 - 5.6,6.1 - 6.6) einen ersten Schenkel (9.1), einen zweiten Schenkel (9.2) und eine sich zwischen diesen Schenkeln (9,1,9.2) erstreckenden Scheitelabschnitt (9.3) umfasst, die sich überlappenden Krümmungsbögen (6.1, 5.1, 6.3, 5.2) im Überlappungsbereich einen gemeinsamen Filamentabschnitt (7) aufweisen, der die Verbindungsstelle (3) bildet, der gemeinsame Filamentabschnitt (7) im Überlappungsbereich in einem Winkel (α) von größer 0°, bevorzugt 10°, relativ zu einer Parallelen der Stützprothesenhauptachse (A) geneigt ist und die gemeinsamen Filamentabschnitte (7) der Überlappungsbereiche in einer flachen Abwicklung der Stützprothese (1) auf der einen Stirnseite eines Stützrings (2.2) in die entgegensetzte Richtung zu den Überlappungsbereichen auf der anderen Stirnseite desselben Stützrings (2.2) geneigt sind, **dadurch gekennzeichnet, dass** sich im Überlappungsbereich neben dem Scheitelabschnitt (9.3) auch Teile der Schenkel (9.1,9.2) der Krümmungsbögen (6.1, 5.1, 6.3, 5.2) überlappen.

2. Stützprothese (1) nach Anspruch 1, **dadurch gekennzeichnet, dass** eine umlaufende Ringmittellinie (M) eines Stützrings (2.1 - 2.7) im wesentlichen in einer senkrecht zur Stützprothesenhauptachse (A) verlaufenden Querschnittsebene angeordnet ist.

3. Stützprothese (1) nach Anspruch 1 oder 2, **dadurch gekennzeichnet, dass** die Mäanderform des Filaments (4) eines Stützrings (2.1 - 2.7) mindestens zwei unterschiedliche Krümmungsbogenformen bzw. -größen aufweist, die gemeinsam ein wiederkehrendes Muster der Mäanderform bilden.

4. Stützprothese (1) nach Anspruch 1 bis 3, **dadurch gekennzeichnet, dass** ein zwischen zwei miteinander verbundenen Überlappungsbereichen gebildetes Prothesenfenster (8) eine erste von dem ersten Stützring (2.1) gebildete Rahmenhälfte (8.1) und eine zweite von dem benachbarten zweiten Stützring (2.2) gebildete Rahmenhälfte (8.2) aufweist, wobei beide Rahmenhälften (8.1, 8.2) punktsymmetrisch zueinander ausgestaltet sind.

5. Stützprothese (1) nach einem der Ansprüche 1 bis 4, **dadurch gekennzeichnet, dass** der Winkel (α) bei mindestens einem Überlappungsbereich 20 bis 30° und bei einem anderen Überlappungsbereich 150 bis 160° beträgt.

6. Stützprothese (1) nach einem der Ansprüche 1 bis 4, **dadurch gekennzeichnet, dass** der Winkel (α) bei mindestens einem Überlappungsbereich 15 bis 25° und bei den anderen Überlappungsbereichen 155 bis 165° beträgt.

7. Stützprothese (1) nach einem der vorangegangenen Ansprüche, **dadurch gekennzeichnet, dass** die gemeinsamen Filamentabschnitte (7) der Überlappungsbereiche zwischen zwei benachbarten Stützringen (2.1,2.2) sämtlich in die gleiche Richtung geneigt sind.

8. Stützprothese (1) nach einem der vorangegangenen Ansprüche, **dadurch gekennzeichnet, dass** ein Stützring (2.1) umlaufend abwechselnd auf der einen Seite mit einem benachbarten Stützring (2.2) und auf der anderen Seite mit einem weiteren benachbarten Stützring (2.3) verbunden ist.

9. Stützprothese (1) nach einem der vorangegangenen Ansprüche, **dadurch gekennzeichnet, dass** das Verhältnis (V) von der Länge (L_{Ü}) des Überlappungsbereichs, in Längsrichtung der Stützprothesenachse (A) gesehen, zur Breite (B₁) des Stützrings (2.1), in Längsrichtung der Stützprothesenachse (A) gesehen, größer ist als 0,1, bevorzugt größer als 0,2.

10. Stützprothese (1) nach einem der vorangegangenen Ansprüche, **dadurch gekennzeichnet, dass** der erste und zweite Schenkel (9.1,9.2) eines Krümmungsbogens (5.1 - 5.6,6.1 - 6.6) in die gleiche Richtung gekrümmt ausgestaltet sind.

11. Stützprothese (1) nach Anspruch 10, **dadurch gekennzeichnet, dass** die ersten und zweiten Schenkel (9.1,9.2) der Krümmungsbögen (6.1 - 6.6) eines Stützrings (2.1) in die gleiche Richtung gekrümmt sind und die ersten und zweiten Schenkel (9.1,9.2) der Krümmungsbögen (5.1 - 5.6) der angrenzenden Stützringe (2.1,2.3) in die entgegengesetzte Richtung gekrümmt sind.

12. Stützprothese (1) nach einem der vorangegangenen Ansprüche, **dadurch gekennzeichnet, dass** auf dem Filament (4) eine Beschichtung (12) vorgesehen ist, die Siliziumdioxid aufweist.

13. Stützprothese (1) nach einem der vorangegangenen Ansprüche, **dadurch gekennzeichnet, dass** jeder Krümmungsbogen (5.1 - 5.6, 6.1 - 6.6) eine Stelle (11) kleinsten Querschnitts aufweist und sich das Filament (4) zumindest abschnittsweise zu der Stelle (11) kleinsten Querschnitts hin kontinuierlich verjüngt.

## Claims

1. Tubular support prosthesis (1) for vessels or intercorporeal lumina, comprising at least two expandable support rings (2.1 - 2.7, 10.1, 10.2) arranged adjacently along a support prosthesis axis (A) and connected to one another at least at one joint (3), said support rings being formed by a filament (4) wound in a meander-like manner to form several arcs of curvature (5.1 - 5.6, 6.1 - 6.6), wherein at least one arc of curvature (6.1) of a first support ring (2.2) and one arc of curvature (5.1) of an adjacent second support ring (2.3) overlap laterally in circumferential direction, and at least one arc of curvature (6.3) of the first support ring (2.2) and one arc of curvature (5.2) of an adjacent third support ring (2.1) overlap laterally in circumferential direction, and a joint (3) is formed in each overlapping portion, each arc of curvature (5.1 - 5.6, 6.1 1 - 6.6) comprises a first leg (9.1), a second leg (9.2) and an apex portion (9.3) extending between these legs (9, 1, 9.2), the overlapping arcs of curvature (6.1, 5.1, 6.3, 5.2) in the overlapping portion have a common filament section (7) forming the joint (3), the common filament section (7) is inclined in the overlapping portion at an angle (α) of greater than 0°, preferably 10°, relative to a parallel of the support prosthesis main axis (A), and the common filament sections (7) of the overlapping portions are inclined at a flat unrolling of the support prosthesis (1) on the one front end of a support ring (2.2) in the opposite direction to the overlapping portions on the other front end of the same support ring (2.2), **characterized in that** beside of the apex section (9.3) also parts of the legs (9.1, 9.2) of the arcs of curvature ( 6.1, 5.1, 6.3, 5.2) overlap in the overlapping portion.

2. Support prosthesis (1) as claimed in claim 1, **characterized in that** a circumferential annular centerline (M) of a support ring (2.1 - 2.7) is substantially arranged in a cross-sectional plane extending perpendicular with respect to the support prosthesis main axis (A).

3. Support prosthesis (1) as claimed in one of the preceding claims 1 or 2, **characterized in that** the meander shape of the filament (4) of a support ring (2.1 - 2.7) comprises at least two different arc of curvature shapes or sizes, which commonly form a recurrent pattern of the meander shape.

4. Support prosthesis (1) as claimed in one of claims 1 to 3, **characterized in that** a prosthesis window (8) formed between two overlapping portions connected to one another comprises a first frame half (8.1) formed by the first support ring (2.1) and a second frame half (8.2) formed by the adjacent second support ring (2.2), wherein both frame halves (8.1, 8.2) are designed in a manner point symmetrical with respect to one another.

5. Support prosthesis (1) as claimed in one of claims 1 to 4, **characterized in that** the angle (α) is 20 to 30° at at least one overlapping portion and 150 to 160° at another overlapping portion.

6. Support prosthesis (1) as claimed in one of claims 1 to 4, **characterized in that** the angle (α) is 15 to 25° at at least one overlapping portion and 155 to 165° at another overlapping portion.

7. Support prosthesis (1) as claimed in one of the preceding claims, **characterized in that** the common filament sections (7) of the overlapping portions are all inclined in the same direction between two adjoining support rings (2.1, 2.2).

8. Support prosthesis (1) as claimed in one of the preceding claims, **characterized in that** a support ring (2.1) is circumferentially alternately connected on the one side with an adjoining support ring (2.2) and on the other side with a further adjoining support ring (2.3).

9. Support prosthesis (1) as claimed in one of the preceding claims, **characterized in that** the ratio (V) of length (L_{ü}) of the overlapping portion, seen in the longitudinal direction of the support prosthesis axis, to the width (B₁) of the support ring (2.1), seen in the longitudinal direction of the support prosthesis axis (A) is larger than 0.1, preferably larger than 0.2.

10. Support prosthesis (1) as claimed in one of the preceding claims, **characterized in that** the first and the second leg (9.1, 9.2) of an arc of curvature (5.1 - 5.6, 6.1 - 6.6) are designed in a manner curved in the same direction.

11. Support prosthesis (1) as claimed in claim 10, **characterized in that** the first and second legs (9.1, 9.2) of the arcs of curvature (6.1 - 6.2) of a support ring (2.1) are curved in the same direction and the first and second legs (9.1, 9.2) of the arcs of curvature (5.1 - 5.6) of the adjoining support rings (2.1, 2.3) are curved in the opposite direction.

12. Support prosthesis (1) as claimed in one of the preceding claims, **characterized in that** a coating (12) is provided on the filament (4), said coating comprising silicon dioxide.

13. Support prosthesis (1) as claimed in one of the preceding claims, **characterized in that** each arc of curvature (5.1 - 5.6, 6.1 - 6.6) comprises a portion (11) of the smallest cross section and the filament (4) continuously tapers at least section-wise towards the portion (11) of the smallest cross section.

## Revendications

1. Endoprothèse tubulaire (1) pour des vaisseaux ou des cavités intracorporelles, avec au moins deux bagues de support expansibles (2.1-2.7, 10.1, 10.2) qui sont placées côte à côte, le long d'un axe d'endoprothèse (A), qui sont reliées entre elles au niveau d'au moins un point de liaison (3) et qui sont formées chacune par un filament (4) enroulé suivant une forme sinueuse pour former plusieurs courbes (5.1-5.6, 6.1-6.6), étant précisé qu'au moins une courbe (6.1) d'une première bague de support (2.2) et une courbe (5.1) d'une deuxième bague de support (2.3) voisine se chevauchent latéralement, dans le sens circonférentiel, et qu'au moins une courbe (6.3) de la première bague de support (2.2) et une courbe (5.2) d'une troisième bague de support (2.1) voisine se chevauchent latéralement dans le sens circonférentiel, que le point de liaison (3) est formé dans chaque zone de chevauchement, que chaque courbe (5.1-5.6, 6.1-6.6) comprend une première branche (9.1), une seconde branche (9.2) et une partie formant sommet (9.3) qui s'étend entre ces branches (9.1, 9.2), que les courbes (6.1, 5.1, 6.3, 5.2) qui se chevauchent présentent dans la zone de chevauchement une partie de filament commune (7) qui forme le point de liaison (3), que la partie de filament commune (7) dans la zone de chevauchement est inclinée suivant un angle (α) supérieur à 0°, de préférence de 10°, par rapport à une ligne parallèle à l'axe principal (A) de l'endoprothèse, et que les parties de filament communes (7) des zones de chevauchement, sur une projection développée plate de l'endoprothèse (1), sont inclinées sur un côté frontal d'une bague (2.2) dans le sens opposé par rapport aux zones de chevauchement situées de l'autre côté frontal de la même bague (2.2),
**caractérisée en ce que** dans la zone de chevauchement située près de la partie formant sommet (9.3), des parties des branches (9.1, 9.2) des courbes (6.1, 5.1, 6.3, 5.2) se chevauchent aussi.

2. Endoprothèse (1) selon la revendication 1, **caractérisée en ce qu'**un axe médian circulaire (M) d'une bague de support (2.1-2.7) est disposé globalement dans un plan en coupe transversale perpendiculaire à l'axe principal (A) de l'endoprothèse.

3. Endoprothèse (1) selon la revendication 1 ou 2, **caractérisée en ce que** la forme sinueuse du filament (4) d'une bague de support (2.1-2.7) présente au moins deux formes ou tailles de courbes différentes qui forment ensemble un modèle de forme sinueuse qui se répète.

4. Endoprothèse (1) selon l'une des revendications 1 à 3, **caractérisée en ce qu'**une fenêtre (8) définie entre deux zones de chevauchement reliées entre elles présente une première moitié de cadre (8.1) formée par la première bague de support (2.1), et une seconde moitié de cadre (8.2) formée par la seconde bague de support (2.2), voisine, les deux moitiés de cadre (8.1, 8.2) ayant une forme symétrique par rapport à un point.

5. Endoprothèse (1) selon l'une des revendications 1 à 4, **caractérisée en ce que** l'angle (α) est de 20 à 30° pour au moins une zone de chevauchement, et de 150 à 160° pour une autre zone de chevauchement.

6. Endoprothèse (1) selon l'une des revendications 1 à 4, **caractérisée en ce que** l'angle (α) est de 15 à 25° pour au moins une zone de chevauchement, et de 155 à 165° pour les autres zones de chevauchement.

7. Endoprothèse (1) selon l'une des revendications précédentes, **caractérisée en ce que** les parties de filament communes (7) des zones de chevauchement entre deux bagues de support voisines (2.1, 2.2) sont toutes inclinées dans le même sens.

8. Endoprothèse (1) selon l'une des revendications précédentes, **caractérisée en ce qu'**une bague de support (2.1), sur sa circonférence, est reliée en alternance à une bague de support voisine (2.2), d'un côté, et à une autre bague de support voisine (2.3), de l'autre côté.

9. Endoprothèse (1) selon l'une des revendications précédentes, **caractérisée en ce que** le rapport (V) de la longueur (L_{Ü}) de la zone de chevauchement, dans le sens longitudinal de l'axe (A) de l'endoprothèse, sur la largeur (B₁) de la bague de support (2.1) dans le sens longitudinal dudit axe (A) est supérieur à 0,1, de préférence supérieur à 0,2.

10. Endoprothèse (1) selon l'une des revendications précédentes, **caractérisée en ce que** les première et seconde branches (9.1, 9.2) d'une courbe (5.1-5.6, 6.1-6.6) sont courbées dans le même sens.

11. Endoprothèse (1) selon la revendication 10, **caractérisée en ce que** les premières et les secondes branches (9.1, 9.2) des courbes (6.1-6.6) d'une bague de support (2.1) sont courbées dans le même sens, et les premières et les secondes branches (9.1, 9.2) des courbes (5.1-5.6) des bagues de support adjacentes (2.1, 2.3) sont courbées dans le sens opposé.

12. Endoprothèse (1) selon l'une des revendications précédentes, **caractérisée en ce qu'**il est prévu sur le filament (4) un revêtement (12) qui contient du dioxyde de silicium.

13. Endoprothèse (1) selon l'une des revendications précédentes, **caractérisée en ce que** chaque courbe (5.1-5.6, 6.1-6.6) présente un point (11) à section transversale minimale, et le filament (4) va en s'effilant de manière continue, au moins par zones, jusqu'au point (11) à section transversale minimale.
